# EUROPEAN PATENT APPLICATION

(11) **EP 3 643 179 A1**
(43) Date of publication of application: **29.04.2020**
(21) Application number: 18887208.9
(22) Date of filing: 13.09.2018
(51) Int. Cl.: A23C 9/20, A23L 33/00

(54) **NUTRITIONAL FORMULA MILK POWDER FOR CONGENITAL HEART DISEASE SURGERY AND NON-CARDIAC SURGERY AND OTHER INTENSIVE CARE INFANTS, AND USE METHOD THEREOF**

(71) Applicant: Original Biological Technology Co., Ltd., Liangxiang, Beijing 102488 (CN)
(72) Inventor: MA, Zeming, Beijing 100020 (CN)
(74) Representative: Kayahan, Senem
(86) International application number: PCT/CN2018/105382
(87) International publication number: WO 2020/051817

(57) **Abstract**

A nutrition formula milk powder for infants after congenital heart disease surgery or non-cardiac surgery and other critically ill infants in intensive care, wherein the milk powder has an energy to protein ratio of 55-60 kcal: 3-4.0g/100 ml.

## Description

### Field of the Invention

The present invention relates to food for special medical purpose, and particularly to nutrition formula milk powder for infants after congenital heart disease surgery or non-cardiac surgery and other critically ill infants in intensive care.

### Background of the Invention

At present, the milk powder available in the market belongs to "formula milk powder" formulated by a scien-tech method, i.e., it is made by using milk as raw material and adding some materials to or extracting some materials from the milk.

Most children patients with congenital heart diseases undergo cardiopulmonary bypass surgery during infancy. Most of these little infants have a low nutritional status and insufficient energy and protein reserves before surgery. Many of children facing other surgery and being intensive care units are also in a low nutritional status when they are admitted to the hospital. After the operation and during stay in the intensive care unit due to critical illness, the children patients are affected by factors such as systemic inflammatory reaction, their catabolism enhances and anabolism reduces, and insufficient nutrition supply worsens malnutrition. Reasonable nutritional feeding after surgery is an important factor affecting recovery. The most important of the nutrients is supply of an appropriate amount of energy and protein, including the absolute amount and ratio of the two. However, due to complex metabolic changes, the design of rational energy and protein nutrition scheme is complex. There is almost no scientific basis for current nutritional feeding schemes. Even nutrition support clinical guidelines for pediatric intensive care published by the most authoritative American Society of Parenteral and Enteral Nutrition and Society of Critical Care Medicine in 2018 also admit that the data is insufficient, and there is very little data after children's heart surgery. At present, in the world there are many milk powder formulas for healthy infants, there are also few formulas which are for special medical purposes and adapted for infants who suffer from lactose intolerance, protein allergy and congenital gastrointestinal tract metabolism abnormity, and there are also high-energy high-protein formulas for infants with low birth weight. However, milk powder for infants after congenital heart disease surgery and infants under intensive care for other critical diseases still remains blank, and such milk powder is characterized by reduced demands for energy and increased demands for protein.

At present, milk powder formulas in the children's world are mostly those for feeding healthy infants. The ratio of energy to protein in all formulas is not suitable for infants after congenital heart disease surgery or non-cardiac surgery and other infants in intensive care. Take NUTRICIA milk powder product as an example. Nutricia is one of the world's largest children's milk powder brands, belongs to Danone Group of France and is headquartered in the Netherlands. The market share of Nutrilon milk powder designed by Nutricia for healthy infants exceeds 70%. Healthy infant milk powder includes Nutrilon Stage 1 (for 0-6-month-old infants) and Stage 2 (for 6-12-month-old infants) milk powder whose energy-protein ratios are65 kcal: 1.4g and 69 kcal: 2.2g/100 ml standard reconstituted solution, respectively. The company designs a type of milk powder, namely, Infatrini high-energy fortified nutrition infant formulated milk powder, for infants with low weight or critical diseases. The milk powder has an energy-to-protein ratio of 100kcal: 2.6g/100ml. The product has been recommended and applied to infants after congenital heart disease surgery or non-cardiac surgery or other critical diseases, but it is still in the research stage. However, for infants developing congenital heart disease and other critical diseases, although most of them can tolerate enteral feeding, energy of Nutrilon is slightly high, but its protein is seriously insufficient; energy of Inatrini is by far higher than the demand, but its protein is still insufficient. Again for example, preNan milk powder designed by Nestle of Switzerland for premature infants and infants born with low body weight has an energy-to-protein ratio of 82kcal: 2.3g/100ml. Its energy is higher than the demand of critical infants, but its protein is still insufficient.

**Ingredients of known milk powder formulas such as Infatrini high-energy fortified nutrition infant formulated milk powder are as follows:**

| **Ingredient: unit** | **/100ml standard reconstituted solution** |
|---|---|
| Energy: kcal | 100 |
| Protein: g | 2.6 |
| Carbohydrate: g | 9.9 |
| Fat: g | 5.4 |
| Dietary fiber: g | 0.6 |
| Vitamin A: ug RE | 83 |
| Vitamin A: | 2 |
| Vitamin E: mg a-TE | 1.1 |
| Vitamin K1:ug | 5.9 |
| Vitamin B1: ug | 150 |
| Vitamin B2: ug | 150 |
| Nicotinic acid: ug | 1200 |
| Pantothenic acid: ug | 500 |
| Vitamin B6: ug | 60 |
| Folic acid: ug | 15 |
| Vitamin B12: ug | 0.41 |
| Vitamin: ug | 2. 3 |
| Vitamin C: ug | 13 |
| Sodium: mg | 4 2 |
| Potassium: mg | 100 |
| Chlorine: mg | 62 |
| Calcium: mg | 101 |
| Phosphorus: mg | 57 |
| Magnesium: mg | 7.5 |
| Iron: mg | 1 |
| Zinc: mg | 0.9 |
| Copper: mg | 60 |
| Manganese: ug | 50 |
| Selenium: ug | 2.5 |
| Iodine: ug | 16 |
| Choline: ug | 14 |
| Inositol: mg | 25 |
| Taurine: mg | 6.9 |
| Nucleotide: mg | 4.3 |

### Summary of the Invention

Optimal demands and ratios of energy and protein for infants (including non-cardiac surgery, trauma, sepsis, etc.) in the comprehensive pediatric intensive care units are obtained by searching for studies on nutrition of critically ill children from world documents.

Ratios of energy and protein needed by infants after congenital heart disease surgery are obtained according to scientific data on the directly-measured actual demands of energy and protein.

The energy and protein metabolism characteristics of two groups of infant patients were similar, and the energy and protein demands are also similar, namely, low energy and high protein.

Therefore, the present invention provides nutrition formula milk powder for infants after congenital heart disease surgery or non-cardiac surgery and other critically ill infants in intensive care, the milk powder having an energy to protein ratio of 55-60 kcal: 3-4.0 g/100 ml.

Optimally, the energy to protein ratio is: 55 kcal: 4 g / 100 ml.

Preferably, the milk powder is used for infants after cardiopulmonary bypass surgery and after non-heart surgery and other critically ill infants.

Optimally, the milk powder starts to be fed after a period of 6-24 hours after the infant's cardiopulmonary bypass surgery, or within 24 hours after non-cardiac surgery or after admission of other critically ill infants to the hospital.

Preferably, the milk powder starts to be fed at 6 hours after the infant's cardiopulmonary bypass surgery, or within 24 hours after non-cardiac surgery or after admission of other critically ill infants to the hospital.

Optimally, the milk powder is used within 5 days after the infant's cardiopulmonary bypass surgery, or within 7-14 days after non-cardiac surgery or after admission of other critically ill infants to an inventive care unit.

Preferably, the infant is 0-2 years old.

Optimally, the baby is 0-12 months old.

Optimally, the baby is under 12 months old.

The present invention further provides a feeding method of the nutrition formula milk powder for infants after congenital heart disease surgery or non-cardiac surgery and other critically ill infants in intensive care. The method recommends beginning to feed the milk powder within 6 hours after surgery, or within 24 hours after non-cardiac surgery or after admission of other critically ill infants to the hospital.

### Detailed Description of Preferred Embodiments

The nutritional status and metabolic changes of infants after cardiopulmonary bypass surgery for congenital heart disease and in other pediatric intensive care units (including non-cardiac surgery, trauma, sepsis, etc.) have many characteristics in common, for example, a rate of occurrence of malnutrition before admission to the hospital is up to 30-50%. During the postoperative period or critical disease, metabolic disorder is caused by the surgery or disease itself, more importantly by systemic inflammatory response, and is characterized by enhanced protein catabolism and reduced anabolism. Proteolysis provides free amino acids that are directed to tissue repair, wound healing and inflammatory responses. This reaction can cause significant muscle loss in critically ill infants. At the same time, energy metabolism and demand also change, and the inflammatory response stimulates increased metabolism, but at the same time, the growth of the infants is inhibited or stopped, and the nutrients for the growing part should not be fed. Therefore, it is very complex to develop a scheme with appropriate energy and protein. Insufficient supply of protein and energy further aggravates muscle loss and aggravates adverse outcomes, and increasing nutrition may reduce the occurrence of adverse outcomes. A goal of proper protein intake of critically ill infants is to maintain protein balance in the event of catabolism caused by stress. In addition to protein intake, energy intake also affects protein balance in critical diseases. However, to date, there are not yet rigorously designed prospective studies that measure critically ill infant's demands for the energy and protein.

According to the recommendations of the World Health Organization (WHO), a method of assessing the demands for protein is to measure the daily nitrogen balance. According to the nitrogen balance method, it is necessary to accurately determine daily nitrogen intake and nitrogen loss, and accurately calculate the situation of balance between the intake and loss. Guidelines for the Provision and Evaluation of Nutrition Support Therapy in the Pediatric Critically Ill Patients published by Society of Critical Care Medicine and American Society of Parenteral and Enteral Nutrition recommend a prescription about measuring resting energy expenditure by indirect calorimetry to determine energy demand and guide daily energy goals. However, due to conceptual and technical limitations, few centers actually measure resting energy expenditure and nitrogen balance. Most centers estimate the demand for energy by using calculation formulas derived from healthy infants. These formulas are not applicable to critically ill infants, which is already known for 20 years.

Although it is generally believed that the nutritional metabolism and utilization of these critically ill infants are different from those of healthy infants, people began to concern the nutrition of critically ill infants in the past 10 years, but evidences for energy and protein demands are still limited, and the optimal supply of energy and protein is still unknown. There is more research on the energy and protein demands of infants in the comprehensive pediatric intensive care unit as compared with cardiopulmonary bypass surgery for congenital heart disease.

The following two parts describe studies of clinical characteristics and nutritional feeding of the two groups of infants. The protein and energy feeding scheme for infants in the comprehensive pediatric intensive care unit is based on a review of results of international comprehensive studies in the past 20 years, while the protein and energy feeding scheme for infants after congenital heart disease is based on measuring the resting energy expenditure daily to guide energy feeding and meanwhile measuring daily nitrogen balance of protein intake groups of different levels, by directly using the internationally first indirect calorimetry method. The metabolic characteristics and document data of the two groups of infant patients are compared, and a new scheme suitable for protein and energy feeding of the two groups of infant patients is obtained.

### 1. Infants in Comprehensive Pediatric Intensive Care Units

For infant patients in the comprehensive pediatric intensive care unit, a ratio of malnutrition upon admission to the hospital is approximately 20-45%. During the stay in the intensive care unit, malnutrition worsens for the above reasons. Malnutrition at admission and during hospitalization critically affected the outcome of the infant patients (including time using a ventilator, stay in the intensive care unit, duration of hospitalization, infection rate during hospitalization, and death rate). Some studies have used indirect calorimetry to measure the resting energy expenditure, with an average range of 55-60 kcal/kg/day. However, due to technical difficulties, the measurement of the resting energy expenditure has not been widely carried out. In contrast, protein demand is less studied. Only a few tests examined the nitrogen balance and energy intake of infant patients in intensive care (see Table 1). In these studies, the protein intake of the high-protein group is 2.8 -3.1 g/kg/day, and the positive nitrogen balance is reached within 5 days after admission to the intensive care unit. Some demands for energy are estimated according to the formula, and some are not considered, but an actual intake is 22-112 kcal/kg/day. To date, there have been no prospective studies to measure the energy and protein demands of critically ill infant patients and to provide nutrients as needed. Chaparro et al. measured the resting energy expenditure and guided energy feeding, and meanwhile measured the nitrogen balance and checked the protein demand. However, the study did not rigorously design a protein feeding scheme, but rather obtained the protein and energy demands based on longitudinal data and regression analysis of clinical feeding during the stay in the intensive care unit. The protein intakes of infant patients are very different, generally low, mostly 0-2g/kg/d, a few reach 3g/kg/d, and very few reach 4g/kg/d. A conclusion thereof is that a minimum protein intake of 1.5 g/kg/d may achieve a zero nitrogen balance with an energy intake of 58 kcal/kg/d at the same time. However, guidelines for nutrition of pediatric critically ill patients published by Society of Critical Care Medicine and American Society of Parenteral and Enteral Nutrition in 2018 clearly indicate that protein intake above this threshold may prevent cumulative negative protein balance and improve prognosis. For critically ill children, the optimal protein intake needed to achieve a positive nitrogen balance might be much higher than this minimum threshold.

The table below is Table 1 which records clinical effects of innovations of the present invention.

**Table 1**

| Study, design, Level/evaluation | Patient case number, people group, age group, supply path | Nutritional intervention | Energy intake (kcal/kg/day) | Protein intake | Protein balance | Other remarkable effects | Limitations |
|---|---|---|---|---|---|---|---|
| | | | | | | | |
| | | | | (g/kg/day) | (g/kg/day) | | |
| Botran et al.performed randomized controlled trial in 2006, with level A/evaluation+ | n=41,73%post-cardiac surgery, intermediate age 7 months (75% <1 year) EN | Protein enrichment more than 5 days VS standard age-applicable formula | The first day, intermediate S, 61.9; intermediate HP, 65.1; The third day, intermediate S, 68.4,; intermediate HP, 74.2; The fifth day 5, intermediate S, 67.5, intermediate HP, 76.6 | The first day, intermediate S, 1.5; intermediate HP, 2.6; The third day, intermediate S, 1.7; intermediate HP, 2.7; The fifth day, intermediate S, 1.5; intermediate HP, 3.1 | The first day, intermediate S, -1.2; intermediate HP, 0.6 The third day, intermediate S, -0.1; intermediate HP-0.2; The fifth day, intermediate S, -0.4; the middle HP, 0.5 | Significantly elevated serum retinol binding protein on the first day and fifth day through a higher protein intae | No measurement or estimation of resting energy expenditure; no blinding; no clinical result |
| Briassoulis et al. performed randomized controlled trial in 2005, with level A/evaluation+ | n=50, respiratory failure, sepsis, critical head injury, 103 (48) months, EN | Enteral feeding, with glutamine, L arginine, omega-3 fatty acids, fiber, vitamin E, beta carotene, Zn, Cu, Se, protein vs standard formula | 5 days later: HP, 58 (35); S, 64 (30) | 5 days later: HP, 2.6 (2); S, 2.2 (0.8) | 5 days later: HP, 0.44 (2.19); S, -0.38 (1.25) | High proportion of diarrhea in HP, high proportion of gastric colonization and abdominal distensionin S | Estimated resting energy expenditure; differences between groups not shown; adding multiple nutrient elements according to an adult formula |
| Briassoulis et al. performed randomized controlled trial in 2005, with level A/evaluation+ | n=40, critical head injury 120 (51) months, EN | Enteral feeding, with glutamine, L arginine, omega-3 fatty acids, fiber, vitamin E, beta carotene, Zn, Cu, Se, protein vs standard | 5 days later, the median, HP, 57; S, 62 | 5 days later, average HP, 2.5; S, 2.2 | 5 days later, intermediate value: HP, 0.44; S, -0.38 | Less positive gastric culture fluid and lower IL-8 levels in HP | Estimated resting energy expenditure; it is unclear whether this is a subgroup of the 2005 study above; adding multiple |
| | | formula | | | | | nutrient elements according to an adult formula |
| Chaloupecky et al. performed randomized controlled trial in 1997, with level A/evaluation+ | n=37, surgical repair of congenital heart disease, 6.7 (3.4) months, PN | Intravenous feeding VS standard IV infusion 1 day after surgery | Non-protein energy kcal; PN33(9); S, 25(15) | PN, 0.8 (0.1); S, 0 | One day after surgery: PN, -0.71 (0.51); S, -1.53 (0.54) | At second day, higher plasma levels of isoleucine, valine, leucine, valine and threonine | No resting energy expenditure was measured or estimated; studies of one day show a little protein intake; only intravenous feeding; no positive balance |
| Van Waardenburg et al. performed randomized controlled trial in 2009, with level A/evaluation+ | n=20,RSV,2.9(1.7) months, EN | Protein-enriched formula more than 5 days VS standard formula | The fifth day: HP, 112(37);S,82(13) | The fifth day: HP2.8 (0.8); S, 1.5 (0.3) | The fifth day: HP, 1.86 (0.73); S, 0.77 (0.46) | Higher plasma matrix and branched chain amino acids in the HP group | No measurement or estimation of resting energy expenditure; small sample; unable to distinguish the effect of protein vs energy when both are increased; No significant clinical results |
| Briassoulis et al. performed randomized controlled trial in 2002, with levelD/evaluation+ | n=71, 25% sepsis, 41% brain damage, 13% respiratory failure, 10% neuromuscular disease, 11% burn, intermediate age 54 months (range, 2-204 months) | Intestinal feeding started within 12 hours after admission to the hospital | Pairing examples: the first day, 22 (9.3); the fifth day, 66 (22.8) | Pairing examples: the first day, 0.69 (0.25); the fifth day, 1.9 (0.59) | Paired samples: the first day -1.63 (1.06); the fifth day, 0.19 (1.06) | On the fifth day, protein intake (RDA) and energy intake (BMR) were positively correlated with NB; MOSF was negatively correlated with NB | Estimate resting energy expenditure; no control group |

| | | | | | | | |
|---|---|---|---|---|---|---|---|
| The relevant information in the above table is: estimated basic metabolic rate; EN, external nutrition; HP, high protein; MOSF, multiple organ system failure; RDA, recommended daily allowance; RSV, respiratory syncytial virus; S, group standard, levels and evaluations are determined by using the 2013 American Dietetic Association Evidence Analysis Manual. All values are shown as mean (SD), unless otherwise noted. | | | | | | | |

Isotope labeling is the most accurate and detailed method for measuring all aspects of protein metabolism (including synthesis, decomposition, and turnover), but the technology is more difficult and rarely used at the centers. According to studies by Dr. Paul Pencharz who is a world-renowned children protein metabolism expert, for infants after non-cardiac surgery, a net protein synthesis rate (i.e., synthesis minus decomposition)of high protein intake (3.9 g/kg/day) is significantly increased relative to low protein group (2.3 g/kg/day). This improvement in nitrogen utilization is primarily achieved by reducing endogenous proteolysis.

In summary, the infants in the comprehensive pediatric intensive care unit need protein 3-4 g/kg/day and energy of 55-60 kcal/kg/day.

In addition, it has been agreed that enteral nutrition is still a preferred manner of nutrition supply. The results of studies published in 2018 showed that starting enteral nutrition early (reach 25-100% of an expected value in 24-48 hours) significantly reduces mortality and hospitalization costs. The latest guidelines for nutrition of pediatric critically ill patients published by Society of Critical Care Medicine and American Society of Parenteral and Enteral Nutrition recommends that it is a proper enteral feeding method to feed early and then gradually increase.

### 2. Infant patients after cardiopulmonary bypass surgery for congenital heart disease

The incidence of congenital heart disease is 6-10% in the world, and the cardiopulmonary bypass surgery is mostly performed during infantry.

Malnutrition in infant patients with congenital heart disease is even more serious. For infant patients with congenital heart disease, preoperative digestive system microcirculation ischemia and hypoxia lead to inadequate nutrient intake and malabsorption, and long-term high metabolic status leads to insufficient energy reserve. The incidence of moderate to critical nutritional imbalance in infant patients under 1 year age may be as high as 50%, and is one of the most common comorbidities. Postoperatively, the systemic inflammatory response caused by cardiopulmonary bypass enhances catabolism and reduces anabolism. In addition, conventionally used vasoactive drugs stimulate energy expenditure, resulting in a significant increase in resting energy expenditure and protein decomposition. On the other hand, current clinical treatment solutions still limit fluid intake and nutrient supply, leading to extremely poor nutritional status in the early postoperative period. It is already known that only five studies involve energy comparisons, i.e., demand and supply. The studies showed that the energy supply was only 7-20% of a required amount within 24 hours after surgery.

There is less research data on protein demands. The guidelines provided by the American Society of Parenteral and Enteral Nutrition recommend that the daily intake of protein for critically ill children is 2-3 g/kg for children 0-2 years old, but also admit lack of adequate data support, especially for children after cardiopulmonary bypass surgery. At present, only three documents have evaluated protein demands of children patients with congenital heart disease in the early period after surgery, but they have not reached a certain conclusion due to the experimental design problems.

In the nutritional schemes of critically ill patients, the current consensus is that enteral feeding is superior to parenteral nutrition, and the nutritional needs are met as early as possible, but actual implementation varies greatly between centers. In Europe, about 30% of children's cardiac care units begin enteral feeding within 12-24 hours after surgery, and 72% of children's cardiac care units begin central venous parenteral nutrition within 1-3 days after surgery. However, more and more evidences support that is safe and effective to start enteral feeding of milk powder as early as possible (6 hours) after surgery.

In summary, in the environment that congenital heart disease treatment technology develop rapidly and various postoperative nutritional treatment guidelines strongly recommend reasonable nutrition therapy, due to technical difficulties and backward ideas, early postoperative nutritional feeding has been stalled for 20 years. The energy and protein demands and feeding manners are still unknown.

The new technique of the present patent application is based on scientific data on resting energy expenditure and nitrogen balance measured simultaneously in the world's first trial, and obtains energy and protein demands of infants in the early period after congenital heart disease surgery, 55kcal/kg/day and 4g/kcal/day, and the ratio of the two, namely, 55kcal: 4g/100ml.

Specifically, the trial is performed by measuring resting energy expenditure daily and delivering energy based thereon within 5 days after surgery of 40 children patients with complex congenital heart diseases. At the same time, these children patients were randomly divided into 3 groups which were respectively given different doses of protein, namely, a controlled group (conventional treatment group) was given protein intake 1.3g/kg/day, a medium-protein group 2.5g/kg/day, a high-protein group 4g/kg/day, and nitrogen balance was measured daily. The protein supply that reaches the positive nitrogen balance at the earliest is the protein demand. Nutrition of all nutritional schemes was fed through the intestinal tract, and the feeding started six hours after the surgery and lasted five days. Results are found in Table 2. The data showed that the high-protein group reached a positive nitrogen balance after surgery on the day of surgery, and decreased on the first day after surgery, namely, a negative nitrogen balance, and became a positive nitrogen balance 2 to 4 days after surgery. The remaining two groups both were in negative nitrogen balance within 5 days. Furthermore, the children patients can tolerate feeding through the intestinal tract. All of these are discovered for the first time in the world.

**Table 2. Comparison of resting energy expenditure, energy intake, and nitrogen balance in three different protein intake groups within 5 days after infant cardiopulmonary bypass surgery**

| | **Time after surgery (day)** | **High-prote in group (14 children patients)** | **Medium-prot ein group (12 children patients)** | **Controlled group (14 children patients)** | **P value** |
|---|---|---|---|---|---|
| Resting energy expenditure | 0 | 65±8 | 61±6 | 61±3 | NS |
| | 1 | 55±7 | 57±5 | 55±2 | |
| (kcal/kg/d) | 2 | 57±8 | 58±6 | 56±5 | |
| | 3 | 54±10 | 56±8 | 55±7 | |
| | 4 | 54±12 | 58±8 | 53±6 | |
| Energy intake | 0 | 40±12 | 35±12 | 35±11 | NS |
| | 1 | 58±15 | 48±20 | 55±15 | |
| | 2 | 58±8 | 60+12 | 54±7 | |
| (kcal/kg/d) | 3 | 55±13 | 59±6 | 59±7 | |
| | 4 | 59±10 | 60+10 | 56±8 | |
| Nitrogen balance | 0 | 90±224 | -60±89 | -364±146 | Pgroup<0.0001 |
| | 1 | -114±230 | -166±127 | -169±120 | Ptime<0.0001 |
| | 2 | 45±161 | -66±175 | -152±144 | Pgroup*time<0.00 01 |
| (mg/kg/d) | 3 | 15±74 | -129±144 | -131±110 | |
| | 4 | 36±131 | -40±181 | -72±107 | |

Wherein, at an infant age of 20 days-600 days, a lower limit of the energy-to-protein ratio is 50:3, an intermediate value of energy-to-protein ratio is 55:4 (an optimal value), an upper limit of the energy-to-protein value is 60:4, and n is the number of patients.

RESULTS: as shown in Table 2, there was no significant difference in the resting energy expenditure and energy intake between the 3 groups during the 5-day trial period (no statistical difference in P values, NS). The nitrogen balance of the three groups increased gradually (Ptime<0.0001), and as the protein intake increased, the nitrogen balance increased significantly within 5 days (Pgroup^{∗}time<0.0001). The high-protein group reached the positive nitrogen balance after surgery on the day of the surgery, and the negative nitrogen balance was observed on the first day after surgery due to an increase in urine output, followed by the positive nitrogen balance in all three days that followed. The remaining two groups were in negative nitrogen balance (Pgroup<0.0001) at all time.

Conclusion: 4g/kg/day is the protein demand. The energy demand was higher after surgery on the day of surgery and 65kcal/kg/d, but only about 40kcal/kg/day (up to 62% of the demand, much higher than the previously reported 7-20%) can be fed in. For the remaining 4 days, the average energy demand is 55kcal/kg/d and is balanced with intake.

Therefore, a reasonable energy-to-protein formula in the milk powder for the infants after the congenital heart disease surgery is the solution proposed by the present invention. Meanwhile, according to the above-mentioned documents published in other critically ill infant research, the energy-to-protein formula of the milk powder is also suitable for infants after non-cardiac surgery and other critically ill infants.

Milk powder formulated based on the scientific data will likely improve early recovery of infants undergoing congenital heart disease surgery and non-cardiac surgery and other critically ill infants. Although currently there is no interventional clinical study of the rational energy protein nutrition scheme for infants after cardiopulmonary bypass surgery for congenital heart disease, observational studies have repeatedly confirmed that malnutrition affects the recovery of critically ill children. A study of 400 children in 31 intensive care units in eight countries found that 30% of children suffer from critical malnutrition on admission to the hospital. The average daily energy and protein supply targets were 64 kcal/kg and 1.7 g/kg, respectively, but actual average intake was only 38% of the target energy and 43% of the target protein amount. Researchers further found that a percentage of the higher target energy fed in the enteral nutrition manner was significantly associated with a lower mortality rate within 60 days (an odds ratio for increasing energy intake from 33.3% to 66.6% was 0.27 (0.11, 0.67), P = 0.002). Another large prospective cohort study of the same research team found that among 1,245 children patients in 59 intensive care units in 15 countries, those children patients with a higher protein intake (60% higher than the target protein 1.9 g/kg/day) had a significantly reduced mortality rate within 60 days than children patients with low protein intake (intake of protein in an enteral nutrition manner is 20% lower than the target) (the odds ratio 0.14 (95% CI: 0.04, 0.52; P = 0.003)). It should be noted that the above two studies have certain limitations, the age span of the children is large (one month to 18 years old), and the energy and protein demands vary greatly with age, so a single energy and protein target intake cannot achieve reasonable feeding. In addition, most of the children in the two experiments were children and school-age children, and children patients without undergoing cardiopulmonary bypass surgery, so the target protein feeding amount is lower.

Protein, carbohydrate and fat are three major nutrients and provide energy. Among them, the energy of protein and carbohydrate is 4 kcal/g, and the energy of the fat is 9 kcal/g. Those skilled in the art can obtain the corresponding formulated milk powder based on the proportion arrangement of the present invention according to the known milk powder formulas or on the basis of the existing milk powder. For example, proportions of ingredients of milk powder are as follows: energy 55 kcal, protein 4g, carbohydrate 5g, and fat 2.3g in every 100ml.

The above embodiments are only preferred embodiments of the present invention. In the technical field, variations and improvements based on technical solutions of the present invention should not be excluded from the protection scope of the present utility model.

## Claims

1. A nutrition formula milk powder for infants after congenital heart disease surgery or non-cardiac surgery and other critically ill infants in intensive care, wherein the milk powder has an energy to protein ratio of 55-60 kcal: 3-4.0 g/100 ml.

2. The nutrition formula milk powder for infants after congenital heart disease surgery or non-cardiac surgery and other critically ill infants in intensive care according to claim 1, wherein the energy to protein ratio is: 55 kcal: 4g/100 ml.

3. The nutrition formula milk powder for infants after congenital heart disease surgery or non-cardiac surgery and other critically ill infants in intensive care according to claim 2, wherein the milk powder is used for infants after cardiopulmonary bypass surgery and non-cardiac surgery and during intensive care.

4. The nutrition formula milk powder for infants after congenital heart disease surgery or non-cardiac surgery and other critically ill infants in intensive care according to claim 3, wherein the milk powder starts to be fed after a period of 6-24 hours after the infant's cardiopulmonary bypass surgery, or within 24-48 hours after non-cardiac surgery or after admission of other critically ill infants to the hospital.

5. The nutrition formula milk powder for infants after congenital heart disease surgery or non-cardiac surgery and other critically ill infants in intensive care according to claim 4, wherein the milk powder starts to be fed within 6 hours after the infant's cardiopulmonary bypass surgery, or within 24 hours after non-cardiac surgery or after admission of other critically ill infants to the hospital.

6. The nutrition formula milk powder for infants after congenital heart disease surgery or non-cardiac surgery and other critically ill infants in intensive care according to claim 5, wherein the milk powder is used within 5 days after the infant's cardiopulmonary bypass surgery, or within 7-14 days after non-cardiac surgery or after admission of other critically ill infants to an inventive care unit.

7. The nutrition formula milk powder for infants after congenital heart disease surgery or non-cardiac surgery and other critically ill infants in intensive care according to claim 6, wherein the infant is 0-2 years old.

8. The nutrition formula milk powder for infants after congenital heart disease surgery or non-cardiac surgery and other critically ill infants in intensive care according to claim 7, wherein the baby is 0-12 months old.

9. The nutrition formula milk powder for infants after congenital heart disease surgery or non-cardiac surgery and other critically ill infants in intensive care according to claim 8, wherein the baby is under 12 months old.

10. A feeding method of the nutrition formula milk powder for infants after congenital heart disease surgery or non-heart disease surgery and other critically ill infants according to any of claims 1-9, wherein the method recommends beginning to feed the milk powder within 6 hours after the congenital heart disease surgery, or within 24 hours after non-cardiac surgery or after admission of other critically ill infants to the hospital.
